# EUROPEAN PATENT APPLICATION

(11) **EP 2 011 866 A1**
(43) Date of publication of application: **07.01.2009**
(21) Application number: 07450118.0
(22) Date of filing: 02.07.2007
(51) Int. Cl.: C12N 15/00, A01K 67/027

(54) **Method for the generation of a non-human animal with an oncogene**

(71) Applicant: Ludwig Boltzmann Gesellschaft GmbH, 1010 Vienna (AT)
(72) Inventor: Eferl, Robert, 1020 Wien (AT); Casanova, Hevia Emilio, 1090 Wien (AT); Stoiber-Sakaguchi, Dagmar, 1080 Wien (AT); Moriggl, Richard, 1190 Wien (AT); Schmid, Johannes, 2340 Mödling (AT); Kenner, Lukas, 1030 Wien (AT); Zenz, Rainer, 1050 Wien (AT); Musteanu, Monica, 1030 Wien (AT); Blaas, Leander, 1080 Wien (AT)
(74) Representative: Sonn & Partner Patentanwälte

(57) **Abstract**

The present invention relates to a method for the generation of a non-human animal with random activation of several oncogenes, comprising
- transfection of an animal or animal cell, preferably a stem cell, with one or more DNA constructs with at least two randomly inducible oncogenes and
- the random induction of one or more of several inactive oncogenes in the animals or animal cells and
- identifying and distinguishing one of the animals or animal cells with one randomly activated oncogene or an activated group of oncogenes from the other randomly generated animals or animal cells with different activated oncogenes.

## Description

The present invention relates to the field of cancer model systems, their construction and use.

Cancer development is a multi-step process. It requires successive mutation events or protein expression changes, each of these with a limited transformation potential. Defined genetic aberrations like a chromosomal translocation in childhood cancer are initiating events. They do not *per se* induce cancer but require cooperative, subsequent mutations, such as gene amplifications or gene-losses. Much is known about the initiating events of cancer development but little knowledge exists about secondary, cooperative mutations that are essential for cancer development. This is mainly due to the lack of suitable mouse models. One "state of the art" approach is the breeding of two transgenic mouse models, each encoding a specific oncogene on the transgene to a compound mouse model that combines both transgenes and thus, allows expression of both oncogenes. Cooperativity of the two oncogenes would accelerate tumorigenesis. However, such "Two-Hit" mouse models are still far away from the multi-hit nature of cancer and do not include the positive selection principle that is fundamental for cancer development.

The WO 2005/020683 describes the tumor models grown from blastocysts with genetic alterations in embryonic stem cells. The alterations comprise inducible knock-in mutations of oncogenes or inducible knock-out mutations of tumor suppressor genes. Different embryonic stem cells with different genetic mutations can be introduced into a blastocyst to obtain a chimeric animal that can express inducible tumors.

It is therefore one goal of the present invention to provide a novel transgenic mouse model.

Therefore, the present invention provides a method for the generation of a non-human animal with an oncogene, comprising
- transfection of an animal or animal cell, preferably a stem cell, with one or more DNA constructs with at least two inducible oncogenes operatively connected with an induction system and
- random induction of the oncogenes which activates either one or more oncogenes of the inducible (inactive)oncogenes in the animal or animal cell and
- identifying and distinguishing one of the animals or animal cells with one randomly activated oncogene or activated group of oncogenes (that give rise to cancer) from the other randomly generated animals or animal cells with different activated oncogenes (that e.g. do not give rise to cancer).

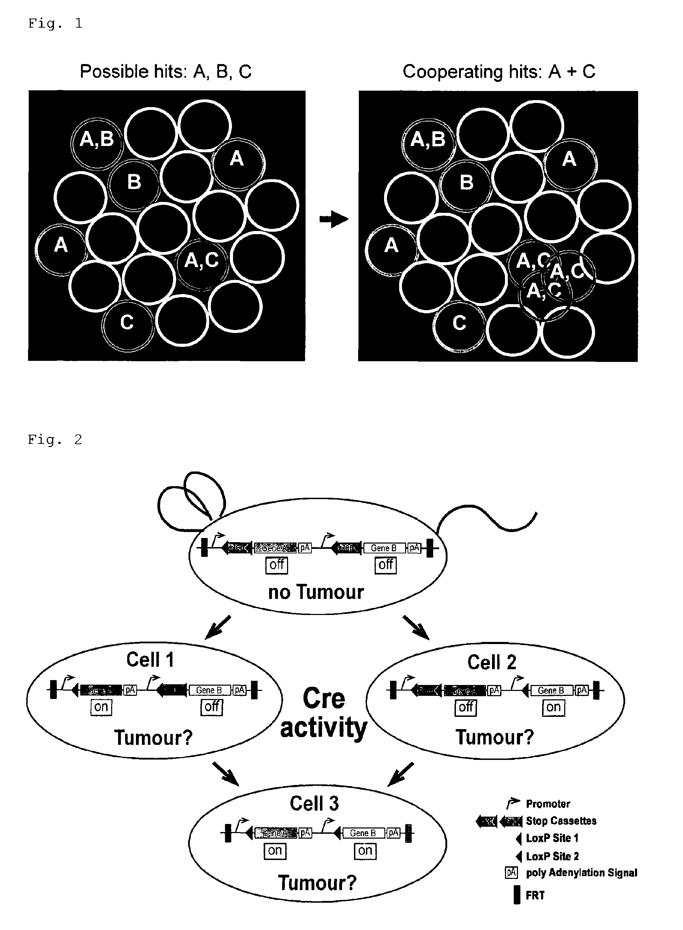

The animal model allows a simultaneous, randomized introduction of several "hits" and is therefore referred to in the present invention as the "Multi-Hit" mouse model. Any combinations of hits that cooperate in tumorigenesis are positively selected and should give rise to a tumor (Figures 1, 2, 3).

Many genetic induction systems are known in the art that can activate certain inducible genes in dependency of endogenous (e.g. tissue specific) or exogenous stimuli (e.g. through contact with chemical stimuli). Such induction systems for the oncogenes are for example recombinase systems such as the cre/lox system, the Flp/FRT or the PhiC31/att system, wherein the recombinase protein is activated in response to an inducible stimulus and in turn leads to activation of the oncogene through recombination at specific recombinase sites (e.g. lox, FRT or att sequences). In preferred embodiments the induction system of at least two oncogenes is the same. The induction randomly activates a first, a second, a third, a fourth, etc. or any combination of oncogenes, thus resulting in many different phenotypes from one originally transfected colony of identical cells or animals. After recombination different cells with multiple activation events may or may not lead to the development of tumors - depending on the cooperative effects of the oncogenes. Thus, the inventive tumor model is an excellent tool to investigate the cooperativity of different oncogenes in tumor formation.

With the term "random induction" it is understood that each inducible oncogene has per se a given (or equal) chance of activation. An oncogene can e.g. be activated to an extent of 0.001% to 90%, in special embodiments to an extent of at least 0.001%, 0.01%, 0.1%, 1%, 2%, 5%, 10%, 15%, 20%, 25%, 30%, 40%, 50% or 75%. The conditions for the activation depend on the induction system and the stimulus selected for the induction system as well as the exposure conditions and time to the stimulus. Conditions are selected where all oncogenes have the chance of being activated. This can e.g. be tested by exposing a multitude of cells, e.g. 10, 100 or 1000, or animals with the stimulus and each possible induction event is observable. E.g. with two oncogenes A + B the resulting cells include cells without activated oncogenes, cells where either A or B is activated and cells with both oncogenes activated. The random induction can be performed in vitro in cells or in vivo in animals that are e.g. grown from genetically modified cells.

The term "oncogene" according to the present invention includes genes which were already associated with tumorigenesis (confirmed oncogenes) but also putative oncogenes which might lead to the formation of tumors alone or in combination with other "oncogenes".

Therefore, the present invention relates to the method for the generation of a non-human animal with a randomly activated combination of candidate genes that are putative oncogenes.

The oncogenes in the animals or cells may be on one DNA construct or introduced by many constructs each with a different oncogene or several oncogenes. Preferably the constructs comprise at least three, preferably at least four, more preferred at least five, randomly inducible oncogenes, wherein the random induction activates either one, two, three, where applicable four or five, oncogenes randomly selected from the oncogenes of the one or more constructs. Of course more oncogenes are possible. In specifically preferred embodiments the oncogenes are on one DNA construct.

In further preferred embodiments the animal cell is a genetically modified embryonic stem cell, which is injected into a blastocyst that is implanted into a surrogate mother, thereby creating a chimeric animal.

Another possibility is to transfect the animal or animal cell with at least one viral vector comprising the one or more DNA constructs. Such viral vectors are for example retroviral, adenoviral or lentiviral vectors, capable of somatic transfection.

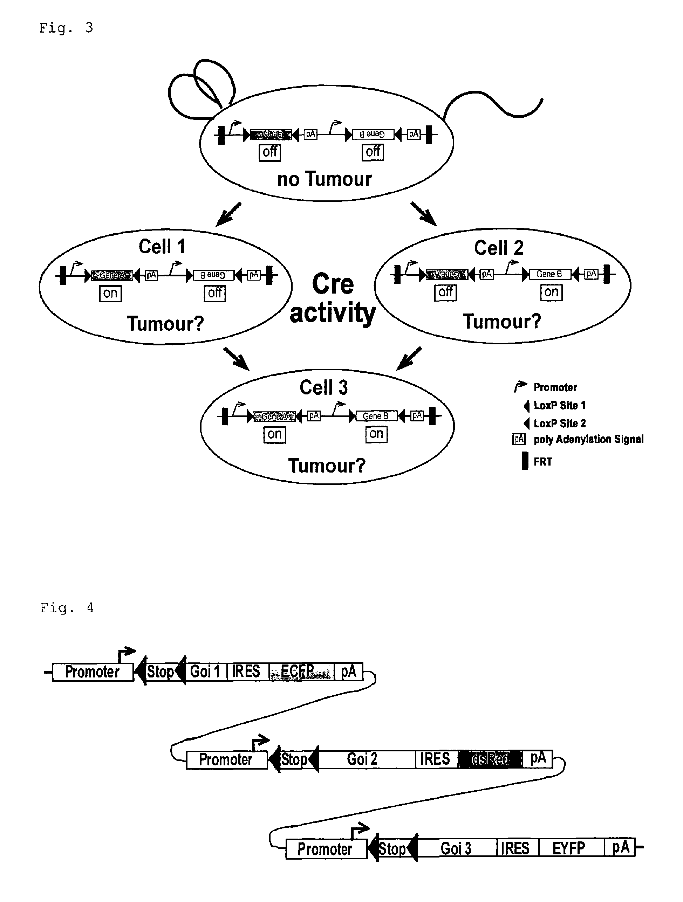

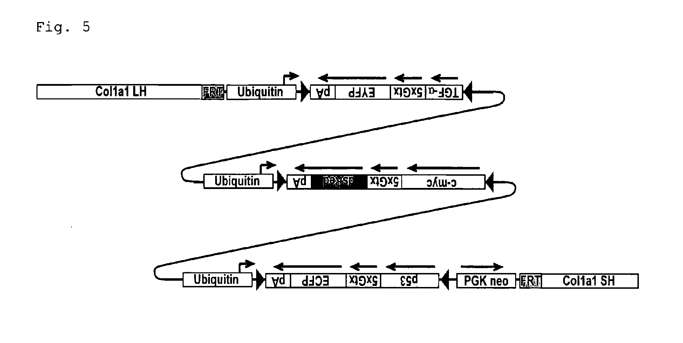

Preferably, the induction leads to the statistical activation of all oncogenes in different animals or cells. In particular all oncogenes are inducible through the same system, e.g. recombinase system in order to guarantee that all oncogenes have the same chance of being activated and investigated through tumor development and activation markers. According to a preferred embodiment, the activation of oncogenes is identified by different marker genes, preferably selected from fluorescent protein marker genes such as GFP, YFP, EGFP, EYFP, CFP or the extracellular domains of human CD2, CD4, CD5 etc. (Figures 4, 5). The marker genes can be located downstream of the oncogene with regard to a promoter on a construct and may be individually translated by introducing an internal ribosomal entry site (IRES) upstream of the marker gene.

Preferably the recombinase system reverses the orientation of the oncogene with respect to the orientation to a promoter (Figures 3, 5). It is possible to place an inactivated oncogene on a construct by inverting the oncogene. Two recombinase sites can be placed flanking the oncogene which - upon activation of the recombinase - can lead to the inversion of the oncogene thus placing it into sense orientation with regard to a promoter.

In other embodiments the recombinase system excises a stop sequence between a promoter and the oncogene (Figures 2, 4). Thus, the promoter can act upon the oncogene and promote transcripton.

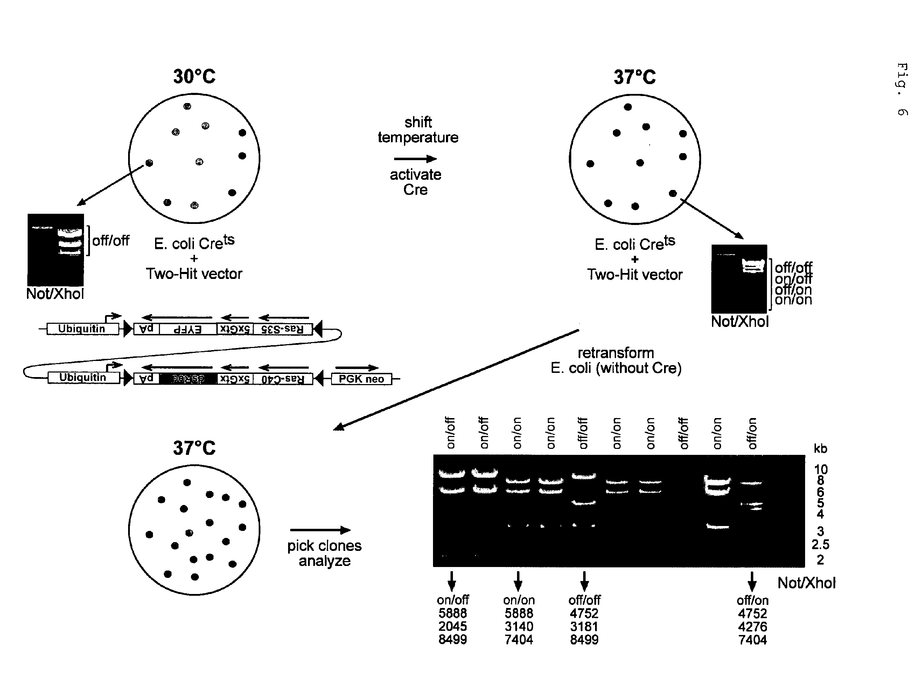

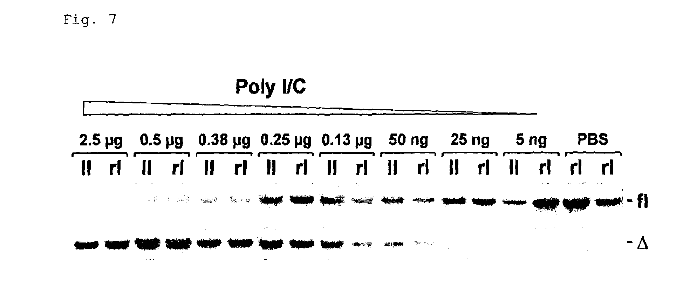

Preferably the random induction is achieved by limited recombinase activity (Figures 6, 7). Limited recombinase activity can be induced in vivo by injection of small amounts of inducing agents (e.g. intraperitoneal injection of small amounts of poly dI/dC into Mx-cre-transgenic mice). An alternative method for in vitro applications is the usage of a temperature sensitive cre recombinase, which is activated by a short temperature shift (e.g. 30 minutes incubation at the permissive temperature).Limited recombinase activity can be induced by titration of inducing agents such as poly dI/dC in the case of the Mx1-cre transgene or Tamoxifen in the case of creER transgenes. Application of these agents at low amounts do not fully activate cre but lead to limited cre induction. Through limited induction preferably less than 100% and up to 0.001% of the oncogenes are activated in the cells or animals.

Preferably, the whole constructs with the oncogenes ("Multi-Hit constructs") after activation by induction of the oncogenes can be excised by a different induction system (different from the induction system used for activation), preferably the Flp/FRT recombinase system. Through inactivation of the initial tumor inducing stimulus (e.g. activation of a combination of oncogenes) it is possible to further characterize the tumors, e.g. if they regress after loss of oncogenic activity. The induction system to be used to, preferably irreversible, remove the oncogenes is a different system than the one used for the activation of the oncogenes but may be selected from the same group of systems.

The animal is especially selected from chicken, fish, fly, worm or mammals, preferably selected from mouse, rat, hamster, sheep, goat or pig. In particular mice are the most preferred animal model, since they are well studied and have a fast growth rate.

In one embodiment of the present invention after the activation of at least one oncogene a tumor is grown, and preferably identified, in the animal.

Another aspect of the present invention provides a DNA construct comprising at least two randomly inducible oncogenes operatively connected to one or more promoter(s), wherein at least two oncogenes are inducible by the same recombinase function, and wherein each oncogene is operatively linked to a unique marker gene, preferably selected from fluorescent protein marker genes.

In preferred embodiments of the present invention the oncogenes are flanked by recombinase sites and the oncogenes are inverted in regard to the promoter. Through activation of a recombinase (e.g. cre, Flp or PhiC31) it is then possible to invert the oncogene through operatively placed recombinase sites, e.g. lox, FRT or att sequences.

In another embodiment of the present invention a stop signal flanked by recombinase sites is positioned between the oncogene and the promoter to interrupt the promoter function for expression of the oncogene. Through recombinase activity the stop signal may be excised thus activating the oncogene.

Preferably, the recombinase is cre and, where applicable, the recombinase sites are lox sites or the recombinase is Flp and, where applicable, the recombinase sites are FRT sites.

In specifically preferred embodiments a DNA stretch comprising at least two oncogenes, preferably all oncogenes, is flanked by recombinase sites, preferably selected from lox sites, FRT sites, preferably different sites as compared to the recombinase sites regulating the individual oncogenes through random induction. This allows the recombinase dependent excision of the oncogenes thus reverting the tumor forming state in a model animal. This different recombinase is inducible by different stimuli than the recombinase for activation of the oncogenes. Preferably, the promoters are upstream of the respective oncogenes.

In a further embodiment of the present invention polyadenylation signals are located downstream of each oncogene in regard to the promoters, preferably, where applicable, before or after the flanking recombinase sites.

The example for a construct with two oncogenes can of course be adapted to constructs with 3, 4, 5, 6, 7, 8, 9, 10 or more oncogenes with different genes of interest with the same recombinase site setup.

In a further aspect, the invention provides a vector comprising a construct according to the present invention, preferably being selected from BAC, PAC, YAC, MAC or viral vectors for somatic transgenesis, preferably retroviral, adenoviral or lentiviral vectors. These vectors can be used to transfect an animal, or an animal cell. It is also possible to transfect bacterial cells for amplification of the construct or recombination.

In another embodiment the present invention provides a non-human animal or animal cell or bacteria comprising one or more DNA constructs at least two randomly inducible oncogenes operatively connected to one or more promoter(s), wherein at least two oncogenes are inducible by the same recombinase function, and wherein each oncogene is operatively linked to a unique marker gene, preferably selected from fluorescent protein marker genes. Such a construct may be as described above and can be introduced by the vector.

Preferably the animal is selected from chicken, fish, fly, worm or mammals, especially mammals being selected from mouse, rat, hamster, sheep, goat or pig.

According to a further aspect, the present invention provides the method of transfecting a non-human animal, preferably being selected from chicken, fish, fly, worm or mammals, especially mammals being selected from mouse, rat, hamster, sheep, goat or pig, comprising the step of introducing a construct of the present invention into a cell of the animal, preferably an embryonic stem cell.

The randomly inducible oncogene can be induced in the animal by limited activation of the recombinase in order to yield an animal wherein one of the at least two oncogenes is activated and an animal wherein the other one of the at least two oncogenes is activated, and preferably one animal wherein two of the at least two oncogenes are activated.

The present invention is further illustrated by the following figures and examples.

### Figures

Figure 1: Principle of the Multi-Hit mouse model. The Multi-Hit mouse model allows the introduction of randomized genetic changes (hits A, B or C) in cells of mouse tissue (e.g. hepatocytes). Only a few cells of the tissue should get a hit (either A, B or C) or a combination of hits (e.g. A + B or A + C). In case that a cell gets a combination of hits that cooperate in tumorigenesis, a tumor would develop (here hits A + C in the right scheme). The hits can then be identified in a tumor biopsy or *in situ.* This mouse model closely mimics the "Darwinian evolution process" of cancer development.
Figure 2: Tumor induction with limited Cre-activity in a Multi-Hit mouse model with two putative oncogenes. The Multi-Hit mouse carries a BAC-transgene with two putative oncogenes (Gene A and B). These genes are not active because of the stop cassettes in front. Induction of limited Cre-activity in the mouse (e.g. by the Mx-cre transgene) will delete the stop cassettes in a subset of cells and generates three different cell types with either one or both oncogenes activated. This mimics the Multi-Hit process of tumor formation and positively selects for tumor formation when the most important oncogenes are activated. In addition, the BAC-transgene is flanked by FRT recombination sites. This will allow deletion of the oncogenes in cultured tumor cells by activation of Flp-recombinase which is important to answer therapeutic questions.
Figure 3: "Flip-Flop" strategy for random activation of putative oncogenes. The oncogenes (A, B) on the BAC-transgene are not active because they are placed in an inverse orientation (written upside down) behind the promoter sequence. The putative oncogenes are flanked by loxP recombination sites (triangles) that are inversely oriented. This prevents deletion but allows a Cre-recombinase-mediated "Flip-Flop" of the oncogenic sequence thereby activating the oncogenes.
Figure 4: A typical Multi-Hit vector used for generation of Multi-Hit transgenic mice. The vector consists of three separate expression units for three different candidate genes or hits (here "genes of interest GOIs" 1, 2 and 3). Transcription starts from a promoter sequence and can be monitored by a reporter gene (here genes for different fluorescent proteins ECFP, dsRed and EYFP). The reporter genes require an internal ribosomal entry site (IRES) and a polyadenylation signal (pA) for expression. The GOIs are kept silent by a stop cassette that is flanked by loxP sites. Mice that are generated with such a Multi-Hit construct can be bred with mice that harbor an inducible cre-transgene. Cre-activation in these double transgenic mice would allow activation of the GOIs. Induction of low Cre-activity would allow deletion of only a few stop cassettes and thereby randomized activation of GOIs.
Figure 5: Scheme of the Multi-Hit vector for the "proof of principle". Two genes that promote liver cancer (TGF-alpha and c-myc) and a gene that antagonizes liver cancer formation (p53) are placed on a Multi-Hit vector. The genes are transcribed from an ubiquitin C promoter but no proteins are generated because the expression modules are cloned in an inverse orientation behind the promoter. Each expression module is flanked by a pair of loxP sites (triangles) that allow Cre-recombinase-mediated inversion (and activation) of the expression modules. The Multi-Hit genes can be randomly activated in liver cells of Multi-Hit mice upon induction of limited Cre-activity (e.g. in Multi-Hit/Mx-cre double transgenic mice) which inverts some modules. The IRES-reporter gene sequences (for fluorescent proteins) allow rapid identification of Multi-Hit gene activation by fluorescence techniques. The whole vector is flanked by homology arms that serve for integration of the Multi-Hit construct into the collagen1a1 locus of embryonic stem cells via homologous recombination. These embryonic stem cells were used for generation of Multi-Hit mice after blastocyst injection. For a combined activation of TGF-alpha and c-myc frequent and big tumors, for individual activation of TGF-alpha or c-myc: rare and small tumors and for activation of p53 no tumors (negative control) were expected.
Figure 6: Random activation of two Multi-Hit genes by gene inversion. *E. coli* cells harboring a temperature-sensitive variant of the Cre-protein were transformed with a vector containing two modules (oncogenic H-Ras genes that selectively activate MAPK and Pi3K signaling pathways) in the "off" (inverted) orientation (see upper left image). Cre-activity in these cells was activated by a temperature shift to 37°C which resulted in a mixture of plasmids with different "off" and "on" orientations of the two modules (upper right image). Re-transformation of the plasmid mixtures into *E. coli* cells without Cre-recombinase and analysis resulted in purified plasmid preparations with genes in the "on/on", "on/off", "off/on" and "off/off" orientations (see lower right image). This example demonstrates that genes can be randomly activated by a short induction of Cre-activity.
Figure 7: Limited and inducible deletion of a floxed sequence (in this case floxed c-jun) in the liver of Mx-cre-transgenic mice. Cre expression was induced with decreasing amounts of poly I/C (per gram of bodyweight) demonstrating a highly reproducible and dose-dependent deletion pattern of the floxed sequence. This example demonstrates that random activation of oncogenes by deletion of stop cassettes is possible in a Multi-Hit mouse model. Shown is the injection of decreasing amounts of Poly I/C into Mx-Cre mice with a floxed allele by Southern Blot for the floxed and the deleted allele with liver DNA. 11: left liver lobe, rl: right liver lobe, fl: floxed band, [delta]: deleted band.

### Examples

### Example 1: Discussion

The Multi-Hit mouse model is technically based on the Cre/loxP site-specific recombination system from the bacteriophage P1. Several gene modules with different candidate genes can be placed on a transgene DNA construct. Each module is a separate expression unit for a candidate gene (see Figures 2 and 4 for details). The candidate genes are not active from the beginning, but can be activated by Cre-activity (the activation of candidate genes is synonymous with mutational hits). We have employed two principles for candidate gene activation. In the first principle, a transcriptional stop cassette is placed between the promoter and the candidate gene (Figures 2, 4). The stop cassette is flanked by loxP sites (floxed) which allows Cre-mediated excision of the stop cassette and thereby activation of the candidate gene. In the second principle, the candidate gene is flanked by loxP sites and placed in an inverse (inactive) orientation behind the promoter (Figures 3 and 5). Cre-activity can invert and thereby activate the candidate gene ("Flip-Flop" principle). The approach is based on random induction of a combination of oncogenes by means of limited recombinase activity. This novel approach can be used to identify essential, cooperating mutations in tumor development because only cells with the relevant, synergistically acting oncogenes will be positively selected, which ultimatively gives rise to tumors (Figures 2 and 3). For randomized gene activation, Multi-Hit mice are injected with transiently acting Cre systems or will be bred with mice that allow induction of Cre-activity (e.g. Mx-cre transgenic mice or tamoxifen inducible Cre transgenic mice) to generate double-transgenic mice.

The expression modules are placed on a novel DNA vector built via modular casette structure that allows expression and detection of the individual modules or selection of the foreign DNA (Figures 4 and 5). The vector DNA is introduced into oocytes for generation of transgenic mice.

The expression modules within the DNA vector can be placed on a bacterial artificial chromosome (BAC). This allows for specific expression patterns and open chromatin structures to better ensure expression of the chosen casettes on such a large DNA. Again, the vector encoded on the BAC can be used for generation of BAC transgenic mice (injection of the BAC into oocytes).

The expression modules and cassettes can also be placed on a DNA vector flanked by homology arms that allow homologous recombination of the construct into a given genomic locus of embryonic stem cells (e.g. the Rosa26 or the collagen 1a1 locus). The corresponding embryonic stem cells can be used for generation of chimaeras and transgenic (or knock-in) mice.

The expression modules can be placed on a BAC and used for homologous recombination of the construct into a given genomic locus of embryonic stem cells (e.g. the Rosa26 or the collagen 1a1 locus). The corresponding embryonic stem cells can be used for generation of chimaeras and transgenic (or knock-in) mice.

The expression modules can be placed on a viral genome (e.g. a retrovirus or a lentivirus) and introduced into mice via viral infection (somatic transgenesis).

### Example 2:

The approach of random gene induction and limited activation of sets of oncogenes ("proof of principle"), i.e. a Multi-Hit mouse model with known outcome is shown in Figure 5. Two genes that promote liver cancer (TGF-alpha and c-myc) and a gene that antagonizes liver cancer formation (p53) are placed on a Multi-Hit vector. The putative oncogenes can be randomly activated in liver cells of transformed mice upon induction of limited Cre-activity (e.g. in Multi-Hit/Mx-cre double transgenic mice) which inverts some modules. It can be expected that liver tumors develop after random activation of the oncogenes. TGF-alpha and c-myc synergize in liver tumorigenesis and should therefore be activated in big tumors. Individual activation of TGF-alpha or c-myc should be observed rarely in small tumors and p53 should not be activated in any tumor (p53 serves as a negative control).

### Example 3:

Random activation of oncogenes by limited Cre-activity in E. coli cells is shown in Figure 6. E. coli cells harboring a temperature-sensitive variant of the Cre-protein were transformed with a vector containing two modules (oncogenic H-Ras genes that selectively activate MAPK and Pi3K signaling pathways) in the "off" (inverted) orientation (see upper left image). Cre-activity in these cells was activated by a temperature shift to 37 °C which resulted in a mixture of plasmids with different "off" and "on" orientations of the two modules (upper right image). Re-transformation of the plasmid mixtures into E. coli cells without Cre-recombinase resulted in purified plasmid preparations with genes in the "on/on", "on/off", "off/on" and "off/off" orientations (see lower right image). This example demonstrates that genes can be randomly activated by a short induction of Cre-activity. In vivo, limited Cre-activity can be achieved by titration of agents that induce expression of cre transgenes or activate CreER fusion proteins. Such an example is shown in Figure 7. Limited and inducible deletion of a floxed sequence (in this case floxed c-jun) in the liver of Mx-cre-transgenic mice. Cre expression was induced with decreasing amounts of polyI/C (per gram of bodyweight) demonstrating a highly reproducible and dose-dependent deletion pattern of the floxed sequence. This example demonstrates that random activation of oncogenes by deletion of stop cassettes is possible in a Multi-Hit mouse model. Shown is the injection of decreasing amounts of Poly I/C into Mx-Cre mice with a floxed allele by Southern Blot for the floxed and the deleted allele with liver DNA.

## Claims

1. Method for the generation of a non-human animal with an oncogene, comprising
- transfection of an animal or animal cell, preferably a stem cell, with one or more DNA constructs with at least two randomly inducible oncogenes operatively connected with an induction system and
- random induction of the oncogenes which activates either one of the at least two oncogenes or at least two oncogenes in the animals or animal cells and
- identifying and distinguishing one of the animals or animal cells with one randomly activated oncogene or activated group of oncogenes from the other randomly generated animals or animal cells with different activated oncogenes.

2. Method according to claim 1, **characterized in that** the induction system of at least two oncogenes is the same.

3. Method according to claim 1 or 2, **characterized in that** the induction system is a recombinase system, preferably the cre/lox system.

4. Method according to any one of claims 1 to 3, **characterized in that** the one or more constructs comprise at least three, preferably at least four, more preferred at least five or more, randomly inducible oncogenes, wherein the random induction activates either one, two, three, where applicable four or five, oncogenes randomly selected from the oncogenes of the one or more constructs.

5. Method according to any one of claims 1 to 4, **characterized in that** the oncogenes are on one DNA construct.

6. Method according to any one of claims 1 to 5, **characterized in that** the animal cell is an embryonic stem cell, which is preferably implanted into a blastocyst.

7. Method according to any one of claims 1 to 6, **characterized in that** the animal or animal cell is transfected with at least one viral vector comprising the one or more DNA constructs.

8. Method according to any one of claims 1 to 7, **characterized in that** the induction leads to the statistical activation of all oncogenes in different animals or cells.

9. Method according to any one of claims 1 to 8, **characterized in that** the activated oncogenes are identified by different marker genes, preferably selected from fluorescent protein marker genes.

10. Method according to any one of claims 3 to 9, **characterized in that** the recombinase system reverses the orientation of the oncogene with respect to the orientation to a promoter.

11. Method according to any one of claims 3 to 9, **characterized in that** the recombinase system excises a stop sequence between a promoter and the oncogene.

12. Method according to any one of claims 3 to 11, **characterized in that** the random induction is achieved by limited recombinase activity.

13. Method according to claims 1 to 12, **characterized in that** at least one activated oncogene is inactivated, preferably by a irreversible recombinase system directed at all oncogenes, more preferred a recombinase system excising the oncogene(s) from the genome of the animal or animal cell.

14. Method according to claim 13, **characterized in that** the oncogenes are inactivated by excision form the construct, preferably by an inducible recombination system different from the induction system for the activation of the oncogenes, preferably the Flp/FRT recombinase system, after random induction.

15. Method according to any one of claims 1 to 13, **characterized in that** the animal is selected from chicken, fish, fly, worm or mammals, preferably selected from mouse, rat, hamster, sheep, goat or pig.

16. Method according to any one of claims 1 to 14, **characterized in that** after the activation of at least one oncogene a tumor is grown, and preferably identified, in the animal.

17. DNA construct comprising at least two randomly inducible oncogenes operatively connected to one or more promoter(s),
wherein at least two oncogenes are inducible by the same recombinase function, and wherein each oncogene is operatively linked to a unique marker gene, preferably selected from fluorescent protein marker genes.

18. Construct according to claim 17, **characterized in that** the oncogenes are flanked by recombinase sites and the oncogenes are inverted in regard to the promoter.

19. Construct according to claim 17, **characterized that** between the oncogene and the promoter a stop signal flanked by recombinase sites is positioned to interrupt the promoter function for expression of the oncogene.

20. Construct according to any one of claims 17 to 19, **characterized in that** the recombinase is cre and, where applicable, the recombinase sites are lox sites.

21. Construct according to any one of claims 17 to 19, **characterized in that** the recombinase is Flp and, where applicable, the recombinase sites are FRT sites.

22. Construct according to any one of claims 17 to 21, **characterized in that** a DNA stretch comprising the at least two oncogenes, preferably all oncogenes, is flanked with recombinase sites, preferably selected from lox sites, FRT sites, preferably different sites as compared to the recombinase sites regulating the individual oncogenes through random induction.

23. Construct according to any one of claims 17 to 22, **characterized in that** the promoters are upstream of the respective oncogenes.

24. Construct according to any one of claims 17 to 23, **characterized in that** polyadenylation signals are located downstream of each oncogene in regard to the promoters, preferably, where applicable, before or after the flanking recombinase sites.

25. Construct according to any one of claims 17 to 24, being **characterized by** a recombinase site map shown in Fig. 3 or Fig. 4.

26. Vector comprising a construct according to any one of claims 17 to 14, preferably being selected from BAC, PAC, YAC, MAC or viral vectors for somatic transgenesis, preferably retroviral, adenoviral or lentiviral vectors.

27. A non-human animal, non-human animal cell or bacteria comprising one or more DNA constructs at least two randomly inducible oncogenes operatively connected to one or more promoter(s), wherein at least two oncogenes are inducible by the same recombinase function, and wherein each oncogene is operatively linked to a unique marker gene, preferably selected from fluorescent protein marker genes.

28. Non-human animal, non-human animal cell or bacteria according to claim 27, **characterized in that** one DNA construct is according to any one of claims 17 to 25.

29. Non-human animal, non-human animal cell or bacteria according to claim 27 or 28, comprising the vector of claim 26.

30. Animal according to any one of claims 27 to 29, being selected from chicken, fish, fly, worm or mammals, preferably selected from mouse, rat, hamster, sheep, goat or pig.

31. The method of transfecting a non-human animal, preferably being selected from chicken, fish, fly, worm or mammals, more preferred selected from mouse, rat, hamster, sheep, goat or pig, comprising the step of introducing a construct according to claims 17 to 25 into a cell of the animal, preferably an embryonic stem cell.

32. The method of inducing a randomly inducible oncogene in an animal according to any one of claims 27 to 30 by limited activation of the recombinase.

33. The method of claim 32, **characterized in that** the recombinase is activated for a short duration in order to yield an animal wherein one of the at least two oncogenes is activated and an animal wherein the other one of the at least two oncogenes is activated, and preferably one animal wherein two of the at least two oncogenes are activated.
